# EUROPEAN PATENT APPLICATION

(11) **EP 1 829 961 A1**
(43) Date of publication of application: **05.09.2007**
(21) Application number: 04807618.6
(22) Date of filing: 22.12.2004
(51) Int. Cl.: C12N 15/00, A01H 5/00, A01K 67/027, A61K 39/395, A61P 9/00, A61P 29/00, A61P 31/04, A61P 31/12, A61P 35/00, A61P 37/00, A61P 37/08, C07K 14/47, C12N 5/00, C12P 21/02, C12P 21/08

(54) **METHOD OF PREPARING ANTIBODY BY USE OF CELL HAVING ITS FUCOSE TRANSPORTER FUNCTION INHIBITED**

(71) Applicant: CHUGAI SEIYAKU KABUSHIKI KAISHA, Tokyo, 115-8543 (JP)
(72) Inventor: TSUCHIYA, Masayuki, c/o Chugai Seiyaku K.K., Gotenba-shi, Shizuoka 412-8513 (JP); IIJIMA, Shigeyuki, c/o Chugai Seiyaku K.K., Gotenba-shi, Shizuoka 412-8513 (JP); SUGO, Izumi, c/o Chugai Seiyaku Kabushiki Kaisha, Gotenba-shi, Shizuoka 412-8513 (JP); SEKIMORI, Yasuo, Chugai Seiyaku Kabushiki Kaisha, Gotenba-shi, Shizuoka 412-8513 (JP); HABU, Kiyoshi, c/o Chugai Seiyaku Kabushiki Kaisha, Gotenba-shi, Shizuoka 412-8513 (JP); SUGIMOTO, Masamichi, Kamakura-shi, Kanagawa 247-8530 (JP)
(74) Representative: Lloyd, John Scott
(86) International application number: PCT/JP2004/019261
(87) International publication number: WO 2006/067847

(57) **Abstract**

The present invention relates to a method of producing a recombinant protein, particularly an antibody, using a cell in which the function of a fucose transporter is inhibited, and it also provides a cell in which the expression of fucose transporter genes on both homologous chromosomes is artificially suppressed.

## Description

### Technical Field

The present invention relates to a method of producing a recombinant protein, particularly an antibody, using a cell in which the function of a fucose transporter is inhibited.

### Background Art

Antibodies can exert anti-tumor effects via their ADCC (antibody-dependent cell-mediated cytotoxicity) activity or CDC (complement dependent cytotoxicity) activity. Antibodies are sugar chain-bound glycoproteins. It is known that an antibody's cytotoxic activity level can vary depending on the types and amounts of sugar chains that bind to the antibody. In particular, it has been reported that the amount of fucose binding to an antibody is strongly involved in the cytotoxic activity level (Shields et al., J Biol Chem., 277(30), 26733-26740, 2002). Furthermore, a method of producing a recombinant antibody not having fucose has been reported. Such method involves preventing an enzyme that catalyzes the binding of fucose to a sugar chain from being expressed upon antibody production in order to obtain an antibody with enhanced cytotoxic activity (International Patent Publication No. WO00/61739).

### Disclosure of the Invention

### Objects to be Achieved by the Invention

An object of the present invention is to provide a method for easily and reliably producing a recombinant protein wherein the binding of fucose is eliminated or decreases. In particular, an object of the present invention is to provide a method of producing an antibody wherein the binding of fucose is eliminated or decreases and whose cytotoxic activity is enhanced. Furthermore, another object of the present invention is to provide a host cell for producing such protein.

### Means to Solve the Problems

In a mechanism by which fucose binds to an antibody within an antibody-producing cell, it is known that GDP binds to fucose that has been incorporated into a cell. GDP-fucose is then incorporated into the Golgi apparatus and then the fucose of the GDP-fucose is transferred to N-acetylglucosamine that has been added as a sugar chain to protein within the Golgi apparatus. Specifically, the Fc region of an antibody molecule has two sites to which an N-glycoside-bound sugar chain binds. Fucose binds to the N-acetylglucosamine portion of an N-glycoside-bound sugar chain (Pate L. Smith et al., J. Cell Biol. 2002, 158, 801-815).

The present inventors have considered that disruption of fucose transporter genes on both chromosomes leads to inhibition of the incorporation of fucose into the Golgi apparatus, so that addition of fucose to an antibody can be inhibited. The present inventors have prepared a cell wherein fucose transporter genes on both chromosomes are disrupted and thus completed the present invention.

In the present invention, to satisfy conditions where the addition of fucose to an antibody is inhibited, it is not necessary that all the produced antibodies do not experience the addition of fucose thereto, but the proportion of protein to which fucose has been added should be decreased among antibody compositions.

The present invention will be described as follows.
[1] A cell, in which the expression of fucose transporter genes on both chromosomes is artificially suppressed.
[2] The cell according to [1], in which the fucose transporter genes are disrupted.
[3] The cell according to [1] or [2], which is an animal cell.
[4] The cell according to [3], in which the animal cell is a Chinese hamster cell.
[5] The cell according to [4], in which the animal cell is a CHO cell.
[6] The cell according to any one of [2] to [5], in which gene disruption is carried out by homologous recombination using a gene targeting vector.
[7] The cell according to any one of [1] to [6], in which a gene encoding a foreign protein is introduced.
[8] The cell according to [7], in which the gene encoding the foreign- protein is a gene encoding an antibody.
[9] A method of producing a protein, comprising culturing the cell according to any one of [1] to [8].
[10] The production method according to [9], in which the protein is an antibody.
[11] The production method according to [10], comprising producing a protein to which fucose is not added.
[12] A method for inhibiting the addition of fucose to a protein, comprising artificially suppressing the expression of fucose transporter genes on both chromosomes upon production of a recombinant protein using a cell.
[13] The method for inhibiting the addition of fucose to a protein according to [12], comprising artificially suppressing gene expression through deletion of a gene.
[14] The method for inhibiting the addition of fucose to a protein according to [12] or [13], in which the protein is an antibody.
[15] The method for inhibiting the addition of fucose to a protein according to any one of [12] to [14], in which the cell is a CHO cell.

### Effect of the Invention

The present invention provides a cell in which the expression of fucose transporter genes on both chromosomes is artificially suppressed. Through the use of the cell for producing a protein, a recombinant protein having no fucose can be produced. Such recombinant protein having no fucose possesses-reduced cytotoxicity and thus is advantageous for use as a recombinant antibody that is used particularly as an antibody drug.

### Brief Description of the Drawings

Fig. 1 shows the structures of 3 types of targeting vector.
Fig. 2 shows the structures of bands that appeared after knockout of the first step and the following cleavage with *Bgl* II.
Fig. 3 shows the results of southern blot analysis in the case of the knockout of the first step.
Fig. 4 shows homologous recombination efficiencies in the case of the knockout of the second step.
Fig. 5 shows the results of Southern blot analysis of fucose transporter gene-deficient cell lines.
Fig. 6 shows the results of fucose expression analysis of a fucose transporter gene-deficient cell line (wild/KO).
Fig. 7 shows the results of fucose expression analysis of a fucose-transporter gene-deficient cell line (KO/KO).

### Preferred Embodiments of the Invention

"Fucose transporter" in the present invention means a polypeptide having fucose transport activity. For example, when a fucose transporter is expressed on the cell membrane, it generally incorporates fucose into the cells. When a fucose transporter is expressed on the Golgi membrane, it generally incorporates fucose into the Golgi apparatus. In the present invention, a preferable fucose transporter is a Chinese hamster fucose transporter, and a more preferable example is a fucose transporter having the amino acid sequence represented by SEQ ID NO: 2. SEQ ID NO: 1 shows the nucleotide sequence of the Chinese hamster fucose transporter gene.

The Golgi apparatus incorporates fucose into itself mainly via fucose transporters existing on the Golgi membrane. Through inhibition of the fucose-transporter function, incorporation of fucose into the Golgi apparatus can be inhibited and the amount of fucose to be incorporated into the Golgi apparatus can be decreased.

To- inhibit the fucose transporter function of cells means to cause a decrease or disappearance of the fucose transport activity of a fucose transporter.

The fucose transporter function of cells may be inhibited by any method. A method for inhibiting fucose transporter expression is preferable.

A method for inhibiting fucose transporter expression is not specifically limited, as long as the number of fucose transporters having normal transport ability decreases. A method that involves deleting (knockout) a gene (fucose transporter gene) encoding a fucose transporter through the use of a targeting vector targeting the fucose transporter or the like is preferable.

Generally a cell has fucose transporter genes on both chromosomes. The cell of the present invention, in which the function of a fucose transporter is inhibited, lacks fucose transporter genes on both chromosomes. Alternatively, the cell of the present invention, in which the function of a fucose transporter- is inhibited, is not particularly limited, as long as it lacks two fucose transporter genes on both chromosomes. Preferably, the cell lacks whole fucose transporter genes existing on chromosomes. For example, when 3 fucose transporter genes in total exist on chromosomes, deletion of 3-fucose transporter genes is preferable. Whether or not all the fucose transporter genes on chromosomes have been deleted can be examined using Southern blot analysis as described in Example below or the FISH method, for example.

Protein produced by the production method of the present invention may be any protein. In general, such protein is a glycoprotein and preferably an antibody.

Types of antibody that are produced by the method of the present invention are not specifically limited. For example, mouse antibodies, rat antibodies, rabbit antibodies, sheep antibodies, camel antibodies, human antibodies, and artificially altered (for the purpose of, for example, lowering heterologous antigenicity against humans) gene recombinant anti-body such as a chimeric antibody or a humanized antibody can be appropriately used. Such gene recombinant antibodies can be produced using a known method. A chimeric antibody comprises the variable region of the heavy and light chains of an antibody of a non-human mammal such as a mouse and the constant region of the heavy and light chains of a human antibody. DNA encoding the variable region of a mouse antibody is ligated to DNA encoding the constant region of a human antibody. The resultant is incorporated into an expression vector, and then the vector is introduced into a host to cause the host to produce the gene product. Thus a gene recombinant antibody can be obtained. A humanized antibody is also referred to as a reshaped human antibody. A humanized antibody is obtained by transplanting the complementarity determining region (CDR) of an antibody of a non-human mammal such as a mouse into the complementarity determining region of a human antibody. General gene recombination techniques therefor are also known. Specifically, DNA sequences designed to have the CDR of a mouse antibody ligated to the framework region (FR) of a human antibody are synthesized by the PCR method from several oligonucleotides, adjacent oligonucleotides of which have an overlap region at their terminal portions. The thus obtained DNA is ligated to DNA encoding the- constant region of a human antibody, the resultant is incorporated into an expression vector, and then the vector is introduced into a host to cause the host to produce the gene product, so that a gene recombinant antibody can be obtained (see European Patent Application Publication No. EP 239400 and International Patent Application Publication No. WO 96/02576). As the FR of a human antibody, which is ligated via CDR, FR that allows the formation of an antigen-binding site with a good complementarity determining region is selected. If necessary, for the formation of an antigen-binding site having the appropriate -complementarity determining region- of a reshaped human antibody, the amino acids- of the framework region of an antibody variable region may be substituted (Sato, K. et al., Cancer Res, 1993, 53, 851-856.). Furthermore, methods for obtaining human antibodies are also known. For example, a desired human antibody having activity of binding to an antigen can also be obtained by sensitizing a human lymphocyte *in vitro* with a desired antigen or a cell that expresses a desired antigen and then fusing the sensitized lymphocyte to a human myeloma cell such as U266 (see JP Patent Publication (Kokoku) No. 1-59878 B (1989)). Moreover, a desired human antibody can be obtained by immunizing a transgenic animal that has all the repertories of a human antibody gene with a desired antigen (see International Patent Application- Publication Nos. WO93/12227, WO92/03918, WO94/02602, WO94/25585, WO96/34096, and WO96/33735). Furthermore, a technique is also known by which a human antibody is obtained by panning using a human antibody library. For example, the variable region of a human antibody is expressed as a single chain antibody (scFv) on the surface of a phage by a phage display method. A phage-that binds to an antigen can be selected. A DNA sequence encoding the variable region of a human antibody that binds to the antigen can be determined by analyzing the gene of the thus selected phage. When the DNA sequence of scFv that binds to an antigen is revealed, an appropriate expression vector is constructed based on the sequence, and then a human antibody can be obtained. These methods are already known. Concerning these methods, WO92/01047, WO92/20791, WO93/06213, WO93/11236, WO93/19172, WO95/01438, and WO95/15388 can be referred to.

Furthermore, the antibody of the present invention may be a lower molecular weight antibody such as an antibody fragment or a modified product of the antibody, as long as such antibody can bind to an antigen. Examples of such antibody fragment include Fab, F(ab')2, Fv, or single chain Fv(scFv) wherein Fv of the H chain and Fv of the L chain are linked using an appropriate linker (Huston, J. S. et al., Proc. Natl. Acad. Sci. U.S.A. (1988) 85, 5879-5883), and a diabody. To obtain such antibody fragment, a gene encoding such antibody fragment is constructed, the gene is introduced into an expression vector, and then the gene is expressed in- an appropriate host cell (e.g., -see Co, M. S. et al., J. Immunol. (1994) 152, 2968-2976; Better, M. and Horwitz, A. H., Methods Enzymol. (1989) 178, 476-496 ; Pluckthun, A. and Skerra, A., Methods Enzymol. (1989) 178, 497-515 ; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137). A diabody is prepared by dimerization; specifically, by linking two fragments (e.g., scFv), each of which is prepared by linking two variable regions using a linker or the like (hereinafter, referred to as a fragment composing a diabody). Generally, a diabody contains two VLs and two VHs (P. Holliger et al., Proc. Natl. Acad. Sci. U.S.A. 90, 6444-6448 (1993); EP404097; WO93/11161; Johnson et al., Methods in Enzymology, 203, 88-98, (1991); Holliger et al., Protein Engineering, 9, 299-305, (1996); Perisic et al., Structure, 2, 1217-1226, (1994); John et al., Protein Engineering, 12(7), 597-604, (1999); Holliger et al., Proc. Natl. Acad. Sci. U.S.A. 90, 6444-6448, (1993); and Atwell et al., Mol. Immunol. 33, 1301-1312, (1996)).

As a modified product of an antibody, antibodies to which various molecules such as polyethylene- glycol (PEG) have been- bound can also- be used. Furthermore, a radioactive isotope, a chemical therapeutic agent, a cytotoxic substance such as toxin derived from bacteria, or the like can be -bound to an antibody. In particular, a radiolabeled antibody is useful. Such modified product of an antibody can be obtained by chemically modifying an obtained antibody. In addition, methods for modifying antibodies have already been established in the field.

### <Protein production method according to the method of the present invention>

A recombinant polypeptide can be produced by a method known by persons skilled in the art. In general, such recombinant polypeptide can be purified and prepared as follows. DNA encoding a polypeptide is incorporated into an appropriate expression vector, a transformant that has been obtained by introducing the vector into an appropriate host cell is collected, and then an extract-is-obtained. Subsequently, the resultant is subjected to chromatography such as ion exchange chromatography, reverse phase chromatography, or gel filtration, affinity chromatography using a column (to which an antibody against the polypeptide of the present invention has been immobilized), or chromatography using combination of a plurality of such columns.

Furthermore, when protein is expressed as a fusion polypeptide with a glutathione-S-transferase protein or as a recombinant polypeptide to which a plurality of histidines have been added in host cells (e.g., animal cells or *Escherichia coli),* the expressed recombinant polypeptide can be purified using a glutathione column or a nickel column. After purification of the fusion polypeptide, if necessary, regions other than the target polypeptide can also be cleaved and removed from the fusion polypeptide using thrombin, factor Xa or the like.

Protein to be produced by the production method of the present invention is preferably an antibody with cytotoxic activity that is affected by fucose binding thereto.

A method of producing an antibody using genetic recombination techniques, which is well known by persons skilled- in the art, involves incorporating an antibody gene into an appropriate vector, introducing the vector into a host, and thus causing the production of the antibody using genetic recombination techniques (e.g., see Carl, A. K. Borrebaeck, James, W. Larrick, THERAPEUTIC MONOCLONAL ANTIBODIES, Published in the United Kingdom by MACMILLAN PUBLISHERS LTD, 1990).

### <Cells to be used in the protein production method of the present invention and protein production using the cells>

Furthermore, the present invention encompasses a host cell that can produce a foreign protein, wherein the expression of fucose transporter genes existing on both chromosomes is artificially suppressed.

A protein having no fucose binding thereto- can be obtained by expressing a foreign- protein using the aforementioned cells wherein the expression of fucose transporter genes on both chromosomes is artificially suppressed as host cells. Here, a foreign protein means a protein not derived from the cell itself. Host cells are not specifically limited. For example, cells wherein sugar is added to a recombinant protein when the protein is expressed can be used. More specifically, various animal cells or the like can be used. Preferably CHO cells can be used. In the present invention, in particular, CHO cells wherein a fucose transporter gene has been knocked out can be appropriately used. As animal cells, mammalian cells such as CHO (J. Exp. Med. (1995) 108, 945), COS, 3T3, myeloma, BHK (baby hamster kidney), HeLa, and Vero are known. As amphibian cells, Xenopus oocytes (Valle, et al., Nature (1981) 291, 358-340), for example, are known. As insect cells Sf9, Sf21, and Tn5, for example, are known. Examples of CHO cells include dhfr-CHO cells (Proc. Natl. Acad. Sci. U.S.A. (1980) 77, 4216-4220) and CHO K-1 cells (Proc. Natl. Acad. Sci. U.S.A. (1968) 60, 1275), which are deficient in a DHFR gene. For the purpose of mass-expression in animal cells, CHO cells are particularly preferable.

Protein having no fucose binding thereto can be obtained by incorporating a gene encoding a foreign protein such as an antibody to be produced into an expression vector and then incorporating the expression vector into host cells capable of producing such foreign protein; that is, cells having an inhibited fucose transporter function. Examples of vectors include expression vectors derived from mammals (e.g., pcDNA3 (produced by Invitrogen Corporation) and pEGF-BOS (Nucleic Acids. Res. 1990, 18(17), p. 5322), pEF, and pCDM8), expression vectors derived -from insect cells (e.g., the "Bac-to-BAC baculovirus expression system" (produced by GIBCO-BRL Life Technologies Inc.) and pBacPAK8), expression vectors derived from plants (e.g., pMH1 and pMH2), expression vectors derived from animal viruses (e.g., pHSV, pMV, and pAdexLcw), expression vectors derived from retroviruses (e.g., pZIPneo), expression vectors derived from yeast (e.g., the "Pichia Expression Kit" (produced by Invitrogen Corporation), pNV11, and SP-Q01), and expression vectors derived from *Bacillus subtilis* (e.g., pPL608 and pKTH50). When CHO cells are used as host cells, it is preferable to use a vector derived from a mammal.

For the purpose of expression in animal cells such as CHO cells, COS cells, or NIH3T3 cells, generally a vector used herein has a promoter required for expression within cells, such as an SV40 promoter (Mulligan et al., Nature (1979) 277, 108), an MMLV-LTR promoter, an EF1α promoter (Mizushima et al., Nucleic Acids Res. (1990) 18, 5322), or a CMV promoter. It is further preferable that such vector has a gene for selection of transformed cells (e.g., a drug resistance gene that enables distinguishment by the use of a drug (e.g., neomycin and G418)). Examples of a vector having such properties include pMAM, pDR2, pBK-RSV, pBK-CMV, pOPRSV, and pOP13.

Furthermore, an example of a method for the purpose of stable expression of a gene and amplification of the number of copies of a gene within cells involves introducing a vector (e.g., pCHOI) having a complementary DHFR gene into CHO cells that are deficient in the nucleic acid synthesis pathway, followed by amplification using methotrexate (MTX). Furthermore, an example of a method for the purpose of transient expression of a gene involves transforming COS cells having a gene that expresses SV40 T antigen on a chromosome with a vector (e.g., pcD) having an SV40 replication origin. As a replication initiation site, a site derived from polyoma virus, adenovirus, bovine papilloma virus (BPV), or the like can also be used. Furthermore, to amplify the number of copies of a gene in a host cell system, an expression vector may contain as a selection marker an aminoglycoside transferase (APH) gene, thymidine kinase (TK) gene, *Escherichia coli* xanthine-guanine phosphoribosyltransferase (Ecogpt) gene, dihydrofolate reductase (dhfr) gene, or the like.

A vector can be introduced into a host cell by, for example, a calcium phosphate method, a DEAE dextran method, a method using cationic ribosome DOTAP (produced by Boehringer Mannheim), an electroporation method, or lipofection.

Cell culture can be -carried out according to a known method. As a culture solution for animal cells, DMEM, MEM, RPMI1640, or IMDM, for example, can be used. At this time, a serum fluid such as fetal calf serum (FCS) can be used together therewith. Alternatively serum-free culture may also be carried out. pH during culture is preferably between approximately 6 and 8. Culture is generally carried out at approximately 30°C to 40°C for approximately 15 to 200 hours. If necessary, exchange of media, aeration, and agitation are carried out.

An example of such a cell wherein the expression of fucose transporter genes on both chromosomes is artificially suppressed is a cell wherein fucose transporter genes existing on both chromosomes have been disrupted.

"Disruption of a gene" means the suppression of the expression of the gene by partial deletion, substitution, insertion, addition, or the like conducted for the nucleotide sequence of the gene. Here, "suppression of gene expression" may also include a case where the gene itself is expressed, -but a gene encoding a protein having normal functions is not expressed.

"Disruption of a gene" of the present invention includes not only a case where gene expression is completely suppressed, but also a case wherein gene expression is partially suppressed. "Deletion (knockout) of a gene" and "inactivation of a gene" are also used so as to have a meaning equivalent to that of "disruption of a gene." Furthermore, cells having a gene disrupted by homologous recombination using gene targeting are referred to as gene-knock out cells. A cell wherein a gene encoding a fucose transporter is disrupted is an example of a cell wherein fucose transporter expression is artificially suppressed.

A cell wherein a gene encoding a fucose transporter is disrupted is a cell wherein the amount of fucose existing in the Golgi apparatus is significantly decreased compared with that in a cell wherein a fucose transporter gene is not disrupted, a cell wherein intracellular fucose transport ability is lowered or deleted, or a cell wherein intracellular activity to incorporate fucose into the Golgi apparatus is lowered or eliminated.

In particular, cells wherein both fucose transporter genes on homologous chromosome pair have been deleted exert the above properties more significantly than cells lacking single fucose transporter gene.

The amount- of fucose -in the Golgi apparatus can be measured by isolating the Golgi apparatus from a cell, extracting sugar, and then carrying out an antigen antibody reaction, a binding reaction between sugar and -lectin, liquid chromatography, electrophoresis, or the like. Moreover, intracellular fucose transport ability and intracellular activity to incorporate fucose into the Golgi apparatus can be determined by, for example, using fucose labeled with a fluorescent substance, a radioisotope, or the like.

A gene can be disrupted-by, for example, a homologous recombination method.

Such homologous recombination method means a method by which only the target gene is arbitrarily altered by homologous gene recombination between a gene on a chromosome and a foreign DNA. Another DNA sequence is inserted into an exon of a gene for the purpose of dividing a sequence encoding a protein. To facilitate identification of a cell having -a gene targeting vector, a selection marker such as a neomycin resistance- gene derived from a bacterium is generally used- as a sequence to divide the gene. A targeting vector is designed and produced based on the sequence information of the fucose transporter gene described in this specification and the fucose transporter gene to be disrupted-is then subjected to homologous recombination using the targeting vector. For example, a substitution vector can contain a homologous region that has been ligated to the 5' and the 3' side of mutation to be introduced, a positive selection marker, a restriction enzyme site for linearizing the vector outside the homologous region, a negative selection marker arranged outside the homologous region, a restriction enzyme cleavage site for detecting mutation, and the like. Targeting vectors can be produced according to methods described in, for example, edited by Kenichi Yamamura et al., Transgenic Animal, KYORITSU SHUPPAN CO., LTD., March 1, 1997; Shinichi Aizawa, Gene Targeting, Production of Mutant Mice using ES cells, Bio Manual Series 8, YODOSHA CO., LTD., 1995; Hogan et al., Manipulating the Mouse Embryo, Cold Spring Harbor Laboratory Press (1944); Joyner, A.L., Gene Targeting, A Practical Approach Series, IRL Press (1993); and edited by Masami Matsumura et al., Experimental Medicine, Separate Volume, New Genetic Engineering Handbook (3rd revised version), YODOSHA CO., LTD., 1999. Both insertion and substitution targeting vectors may be used. Furthermore, recombination can also be caused by targeting using a Cre-lox system. Targeting using the Cre-lox system can be carried out according to a method described in, for example, JP Patent Publication (Kohyo) NO. 11-503015 A (1999). As a method for selecting homologous recombinants that have experienced homologous recombination, a known selection method such as positive selection, promoter selection, negative selection, -or polyA selection may be used. For identification of a homologous recombinant, both the PCR method and the Southern blotting method can be used.

Furthermore, a cell wherein the fucose transporter gene of the present invention is disrupted can also be obtained by randomly introducing a mutation into a cell, as long as both fructose transporters on chromosome pair are deleted. Examples of a method for randomly introducing mutation into a cell include a method that involves randomly introducing a gene disruption vector containing a marker into-the genome of a cell and then screening for a cell having a disrupted fucose transporter gene, and a method that involves randomly introducing mutation using an chemical mutagen such as ENU (N-ethyl-N-nitrosourea) and then screening for such cell having a disrupted fucose transporter gene. Screening for cells that have become unable to produce any fucose transporter may be carried out using fucose transporter activity as an index. Alternatively, such screening can also be carried out by Western blotting or Northern blotting using fucose transporter gene transcription or expression as an index.

Furthermore, the cell of the present invention having a disrupted fucose transporter gene can also be obtained from an animal having a knocked-out fucose transporter gene. Such animal having a knocked-out fucose transporter gene can- be produced by disrupting a fucose transporter of an ES cell by the above-method and then producing from the ES cell according to, for example, a method disclosed in WO02/33054 Publication. Examples of animals that are used in this- case include, but are not limited to, goats, pigs, sheep, cattle, mice, hamsters, and rats. Established cells having no fucose transporter genes can be obtained by producing such established cells from animals having a knocked-out fucose transporter gene.

When a foreign recombinant protein is produced according to the present invention in host cells wherein two fucose transporter genes on both chromosomes have been disrupted, addition of fucose to a protein is inhibited. This is because fucose within each of the cells is not transported into the Golgi apparatus. In the case of such recombinant protein produced in host cells having disrupted fucose transporter genes, the amount of bound fucose is significantly lower or preferably unable to be detected, compared with -the case of recombinant protein -produced in host cells having an undisrupted fucose transporter gene. When a foreign protein is an antibody, a product can be obtained wherein no fucose is binding--to an N-glycoside-bound sugar chain binding to 2 sugar-chain-binding sites existing in 1 molecule of an antibody; that is, existing in= the Fc region composed of 2 H chains. Such antibody having no fucose binding thereto has enhanced cytotoxic activity. In addition, when an antibody for which the addition of fucose thereto is inhibited is produced using the cell of the present invention, it is not necessary that all the produced antibodies have not experienced the addition of fucose thereto. The proportion of protein to which fucose has been added in antibody compositions should be reduced.

Furthermore, the present invention also encompasses animals (excluding humans) wherein fucose transporter genes- on both chromosomes are deleted. A recombinant polypeptide can be produced *in vivo* using such animals.

DNA encoding target protein is introduced into these animals, polypeptides are produced *in vivo* within the animals, and then the polypeptides are collected. The term "host" in the present invention encompasses these animals and the like. When animals are used, there are production systems using mammals and production systems using insects. As mammals, goats, pigs, sheep, mice, or cattle can be used (Vicki Glaser, SPECTRUM Biotechnology Applications, 1993).

For example, a target DNA is prepared in the form of a fusion gene with a gene encoding a polypeptide such as goat β casein that is uniquely produced in milk. Subsequently, a DNA fragment comprising the fusion gene is injected into a goat embryo and then the embryo is transplanted into a female goat. A target polypeptide can be obtained from milk that is produced by transgenic goats born from goats that have accepted such embryos or from the progenies of such transgenic goats. To increase the amount of milk containing polypeptides, which is produced by transgenic goats, an appropriate hormone may also be used for such transgenic goats (Ebert, K.M. et al., Bio/Technology (1994) 12, 699-702).

The thus obtained polypeptide can be isolated from within host cells or outside the cells (e.g., media) and then purified as a substantially pure -and uniform polypeptide. To separate and purify polypeptides, separation and purification methods that are generally used for polypeptide purification may be employed and are not specifically limited. For example, polypeptides can be separated and purified by the use of appropriate selection and combination of a chromatography column, a filter, ultrafiltration, salting-out, solvent precipitation, solvent extraction, distillation, immunoprecipitation, SDS-polyacrylamide gel electrophoresis, an isoelectric focusing method, dialysis, recrystallization, and the like.

Examples of chromatography include affinity chromatography, ion exchange chromatography, hydrophobic chromatography, gel filtration, reverse phase chromatography, and adsorption chromatography (Strategies for Protein Purification and Characterization: A Laboratory Course Manual, Ed Daniel R. Marshak et al., Cold Spring Harbor Laboratory Press, 1996). These types of chromatography can be carried out using liquid-phase chromatography such as HPLC or FPLC.

In addition, when a proper protein modification enzyme is caused to act on polypeptides before or after purification, modification can be arbitrarily carried out or peptides can be partially removed. As protein modification enzymes, trypsin, chymotrypsin, lysylendopeptidase, protein kinase, and glucosidase, for example, are used.

A known sequence can also be used for a gene encoding the H chain or the L chain of an antibody that is produced by the production method of the present invention. Furthermore, such gene can also be obtained by a method known by persons skilled in the art. For example, a gene encoding an antibody can be cloned and obtained from a hybridoma producing a monoclonal antibody or can also be obtained from an antibody library. Hybridomas can be produced basically using a known technique as described below. Specifically, a hybridoma can be produced by using as a sensitizing antigen a desired antigen or a cell that expresses a desired antigen, carrying out immunization according to a general immunization method using such antigen, fusing the thus obtained immunocyte with a known parent cell by a general cell fusion method, and then screening for a monoclonal antibody-producing cell (hybridoma) by a general screening method. The cDNA of an antibody variable region (V region) is synthesized from the mRNA of the thus obtained hybridoma using reverse transcriptase. The cDNA is ligated to DNA encoding a desired antibody constant region (C region), so that a gene encoding the H chain or the L chain can be obtained. A sensitizing antigen upon immunization is not specifically limited. For example, a target full-length protein or partial peptide can be used. Antigens can be prepared by a method known by persons skilled in the art. For example, antigens can be prepared according to a method using baculovirus (e.g., WO98/46777). Hybridomas can be produced according to, for example, Milstein et al's method (Kohler, G., and Milstein, C., Methods Enzymol. 1981, 73, 3-46) or the like. When the immunogenicity of an antigen is low, such antigen is bound to a macromolecule having immunogenicity, such as albumin, and then immunization- is carried out.

Regarding an antibody library, many antibody libraries are already known. In addition, a production method for an antibody library is known. Hence, persons skilled in the art can appropriately obtain an antibody library.

Antibodies that are expressed and produced as described above can be purified by a known method that is used for general protein purification. Antibodies can be separated and purified by appropriate selection or combination of, for example, an affinity column (e.g., protein A column), a chromatography column, a filter, ultrafiltration, salting-out, and dialysis (Antibodies: A Laboratory Manual, Ed Harlow and David Lane, Cold Spring Harbor Laboratory, 1988).

A known means can be used for measuring the antigen-binding activity of an antibody (Antibodies A Laboratory Manual, Ed Harlow, David Lane, Cold Spring Harbor Laboratory, 1988). For example, ELISA (enzyme-linked immunosorbent assay), EIA (enzyme-linked immunoassay), RIA (radioimmunoassay), or a fluorescent immunoassay can-be used.

The sugar chain structure of protein -produced using the cell of the present invention can be analyzed by a method described in a 2-dimensional sugar chain mapping method (Anal. Biochem, 171, 73 (1988); Biochemical Experimental Methods 23-Methods for Studying Glycoprotein Sugar Chains, edited by Reiko Takahashi, Center for Academic Publications Japan (1989)). Moreover, sugar chains can also be analyzed by mass spectrometry such as MALDI-TOF-MS.

### Cytotoxic activity of antibody

Antibodies produced by the method of the present invention have enhanced cytotoxic activity.

Examples of cytotoxic activity in the present invention include antibody-dependent cell-mediated cytotoxicity (ADCC) activity and complement-dependent cytotoxicity (CDC) activity. In the present invention, CDC activity means cytotoxic activity of a complement system. ADCC activity means activity to damage a target cell. Specifically, when a specific antibody attaches to a cell surface antigen of a target cell, an Fcy receptor-retaining cell (e.g., an immunocyte) binds to the Fc portion of the antibody via an Fcγ receptor, damaging the target cell.

Whether or not an antibody has ADCC activity or has CDC activity can be determined by a known method (e.g., Current protocols in Immunology, Chapter 7, Immunologic studies in humans, Editor John E. Coligan et al., John Wiley & Sons, Inc., (1993)).

Specifically, effector cells, a complement solution, and target cells are prepared.
(1) Preparation of effector cells
   A spleen is extracted from a CBA/N mouse or the like, and then spleen cells are separated in an RPMI1640 medium (produced -by GIBCO). After washing with the same medium containing 10% fetal bovine serum (FBS, produced by HyClone), the cells are prepared to a concentration of 5 × 10⁶/ml, thereby-preparing effector cells.
(2) Preparation of a complement solution
   Baby Rabbit Complement (produced by CEDARLANE LABORATORIES LIMITED) is diluted 10-fold in a 10% FBS-containing medium (produced by GIBCO), thereby preparing a complement solution.
(3) Preparation of target cells

Pancreatic cancer cell lines (e.g., AsPC-1 and Capan-2) are radiolabeled by culturing the cell lines with 0.2 mCi ⁵¹Cr-sodium chromate (produced by Amersham Pharmacia Biotech) in a 10% FBS-containing DMEM medium at 37°C for 1 hour. After radiolabeling, the cells are washed- three times in a 10% FBS-containing RPMIL640 medium and then prepared to a cell concentration of 2 × 10⁵/ml, thereby preparing target cells.

Subsequently, ADCC activity or CDC activity are determined. To determine ADCC activity, target cells and antibodies are added in amounts of 50 ml each to a 96-well U-bottomed plate (produced by Becton, Dickinson and Company) and are then allowed to- react on ice for 15 minutes. Next, 100 µl of effector cells is added, followed by 4 hours of culture in a carbon dioxide gas incubator. The final antibody concentration is 0 or 10 µg/ml. After culture, 100 µl of the supernatant is collected, and then radioactivity is measured using a gamma counter (COBRAIIAUTO-GMMA, MODEL D5005, produced by Packard- Instrument Company). Cytotoxic activity (%) can be calculated by (A-C)/(B-C) × 100. "A" denotes radioactivity (cpm) in each sample, "B" denotes radioactivity (cpm) in a sample supplemented with 1% NP-40 (produced by NACALAI TESQUE, INC.), and "C" denotes radioactivity (cpm) in a sample containing only target cells.

Furthermore, to determine CDC activity, target cells and anti-PepT antibodies are added in amounts of 50 µl each to a 96-well flat-bottomed plate (produced by Becton, Dickinson and Company) and are then allowed to react on ice for 15 minutes. Subsequently, 100 µl of a complement solution is added, followed by 4 hours of culture in a carbon dioxide gas incubator. The final antibody concentration is 0 or 3 µg/ml. After culture, 100 µl of the supernatant is collected, and then radioactivity is measured using a gamma counter. Cytotoxic activity can be calculated in a manner similar to that used for determination of ADCC activity.

### Example

### Disruption of a fucose transporter gene in CHO cells

### 1. Construction of targeting vectors

### (1) Preparation of KO1 vector

A hygromycin resistance gene (Hyg^{r}) was prepared by PCR using pcDNA3.1/Hygro (Invitrogen Corporation) as a template and Hyg5-BH and Hyg3-NT as primers. A *Bam*H I and a TGCGC sequence were added to the 5' side of the initiation codon of Hyg^{r}, thereby preparing a sequence that was the same as that on the 5' region of the initiation codon of a fucose transporter gene. A *Not* I sequence was added to the 3' region comprising a SV40 polyA addition signal, thereby extracting Hyg^{r}.
Forward primer
Hyg5-BH 5'- GGA TCC TGC GCA TGA AAA AGC CTG AAC TCA CC -3' (SEQ ID NO: 3)
Reverse primer
Hyg3-NT 5'- GCG GCC GCC TAT TCC TTT GCC CTC GGA-CG -3' (SEQ ID NO: 4)

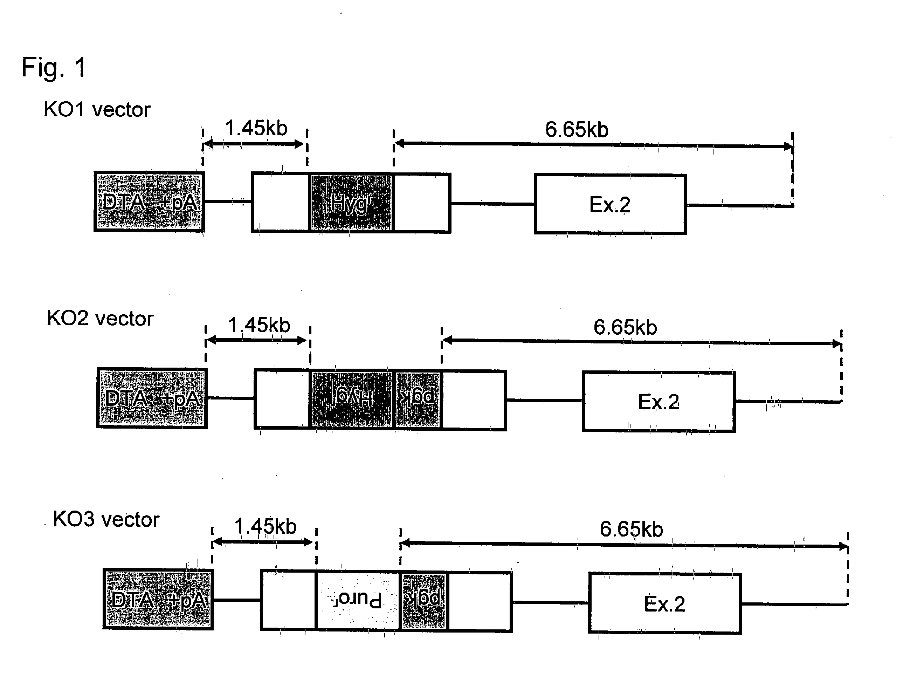

A targeting vector ver. 1 (hereinafter referred to as KO1 vector) for a fucose transporter knockout was constructed by inserting separately a 5' fragment (ranging from No. 2,780 *Sma* I to No. 4,232 *Bam*H I of the nucleotide sequence shown- in SEQ ID NO: 1) of a fucose transporter, a 3' fragment (ranging from No.4,284 to No. 10,934 *Sac* I) of the fucose transporter, and a Hyg^{r} fragment into a pMC1DT-A vector (Yagi T, Proc. Natl. Acad. Sci. U.S.A. vol. 87, pp. 9918-9922, 1990) (Fig. 1). The vector is characterized by carrying promoter-less Hyg^{r} unit. Thus, Hyg^{r} is expressed by a promoter of the fucose transporter following homologous recombination. However, introduction- of only one copy of a vector into cells by homologous recombination does not always result in the enough expression of Hyg^{r} that provide resistance against hygromycin B in the cells. In addition, the KO1 vector was linealized by *Not* I and transfected into cells. With the use of the KO1 vector, the fucose transporter gene will lack 41 base pairs of exon 1 comprising the initiation codon and lose the relevant function.

### (2) Preparation of pBSK-pgk-1-Hyg^{r}

A mouse pgk-1 gene promoter was prepared from a pKJ2 vector (Popo H, Biochemical Genetics vol. 28, pp. 299-308, 1990) by *EcoR* I*-Pst* I digestion and then cloned into the *EcoR* I-*Pst* I site of pBluescript (Stratagene Corporation), thereby preparing pBSK-pgk-1. A hygromycin resistance gene (Hyg^{r}) was prepared by PCR using pcDNA3.1/Hygro (Invitrogen- Corporation) as a template and Hyg5-AV and Hyg3-BH as primers. Thus, an Eco T221 and a Kozak sequence were added to the 5' region of Hyg^{r}. Furthermore, a *BamH* I sequence was added to the 3' region comprising a SV40 polyA addition signal, thereby extracting Hyg^{r}.
Forward primer
Hyg5-AV 5'- ATG CAT GCC ACC ATG AAA AAG CCT GAA CTC ACC -3' (SEQ ID NO: 5)
Reverse primer
Hyg3-BH 5'- GGA TCC CAG GCT TTA CAC TTT ATG CTT C -3' (SEQ ID NO: 6)

The Hyg^{r} (EcoT22 I*-Bam*HI) fragment was inserted into the *Pst*I*-Bam*H I site of pBSK-pgk-1, thereby preparing-pBSK-pgk-1-Hyg^{r}.

### (3) Preparation of KO2 vector

A targeting vector-ver.2 (hereinafter referred- to as the KO2 vector) for a fucose transporter knockout was constructed by- inserting separately a 5' region(ranging from No. 2,780 *Sma* I to No. 4,232 *Bam*H I in the nucleotide sequence shown in SEQ ID NO: 1), a 3' region(ranging from No. 4,284 to No. 10,934 *Sac-*I) of the fucose transporter, and pgk-1-Hyg^{r} fragments into a pMC1DT-A vector (Fig. 1). Unlike the KO1 vector, a pgk-1 gene promoter was added to Hyg^{r} of the KO2 vector. Introduction of even only one copy of a vector into cells by homologous recombination can cause the cells to acquire resistance to hygromycin B. In addition, the KO2 vector was linealized by *Not* I and then transfected into cells. With the use of the KO2 vector, the fucose transporter may lack 46 base pairs of exon 1 comprising the initiation codon and lose the relevant function.

### (4) Preparation of pBSK-pgk-1-Puro^{r}

A pPUR vector (BD Biosciences) was digested with *Pst* I and *BamH* I. The Puro^{r} fragment was inserted into the *Pst* I*-Bam*H I site of pBSK-pgk-1, thereby preparing pBSK-pgk-1-Puro^{r}.

### (5) Preparation of KO3 vector

A targeting vector ver.3 (hereinafter referred to as the K03 vector) for a fucose transporter knockout was constructed by separately inserting a 5' region (ranging from No. 2,780 *Sma* I to No. 4,232 *Bam*H I of the nucleotide sequence shown in SEQ ID NO: 1), a 3' region (ranging from No. 4,284 to No. 10,934 *Sac* I) of the fucose transporter, and pgk-1-Puro^{r} fragments into a pMC1DT-A vector (Fig. 1). In addition, a sequence, to which the following primer for screening binds had been previously added to the 3' end of pgk-1-Puro^{r}. In addition, the KO3 vector was linealized with *Not* I and then transfected into cells. With the use of the KO3 vector, the fucose transporter may lack 46 base pairs of exon 1 comprising the initiation codon and lose the relevant function.
Reverse primer
RSGR-A 5'-GCT GTC TGG AGT ACT GTG CAT CTG C -3' (SEQ ID NO: 7)

Knockout of the fucose transporter gene was -attempted using the above 3 types of targeting vector.

### 2. Vector transfection into CHO cells

HT Supplement (100 ×) (Invitrogen Corporation cat. 11067-030) and penicillin streptomycin (Invitrogen Corporation cat. 15140-122) were each added to CHO-S-SFMII HT-(Invitrogen Corporation cat 12052-098) in a volume one-hundredth of the volume of CHO-S-SFMII HT-. The solution was used as medium for culture (hereinafter referred to as SFMII (+)). The DXB11 cell line of CHO cells was maintained and cells after gene transfer were also cultured in SFMII (+). 8 × 10⁶ CHO cells were suspended in 0.8 mL of Dulbecco's phosphate buffer (hereinafter abbreviated as "PBS"; Invitrogen Corporation cat. 14190-144). 30 µg of the targeting vector was added to the cell suspension. The cell suspension was transferred to a Gene Pulser Cuvette (4 mm) (Bio-Rad Laboratories Inc. cat. 1652088). After the suspension had been allowed to stand on ice for 10 minutes, the cells were electroporated using GENE-PULSER II (Bio-Rad Laboratories Inc. code No. 340BR) at setting of 1.5 kV, 25 µFD. After vector transfection, the cells were suspended- in 200 ml of SFMII(+) medium. The cell suspension was then dispensed into twenty 96-well flat-bottomed plates (IWAKI cat. 1860-096) at 100 µl/well. The cells in the plates were cultured in a CO₂ incubator for 24 hours at 37°C, followed by addition of a drug.

### 3. First step of knockout

The KO1 vector or the KO2 vector was transfected into CHO cells. At 24 hours after vector transfection, selection was performed using hygromycin B (Invitrogen Corporation cat. 10687-O10). Hygromycin B was added to SFMII(+) to a concentration of 0.3 mg/ml and then added to transfected cells at 100 µl/well.

### 4. Screening of homologous recombinants by PCR

### (1) Preparation of samples for PCR

Homologous recombinants were screened by the PCR method. CHO cells to be used in screening were cultured in a 96-well flat-bottomed plate. After removal of culture supernatants, a buffer for cell lysis was added at 50 µl/well. After incubation at 55°C for 2 hours, proteinase K was inactivated by subsequent heating at 95°C for 15 minutes, so as to prepare a template for PCR. The buffer for cell lysis was composed of 5 µl of 10 × LA buffer II (attached to TaKaRa LA Taq), 2.5 µl of 10% NP-40 (Roche cat. 1 332 473), 4 µl of proteinase K (20 mg/ml and TaKaRa cat. 9033), and 38.5 µl of distilled water (Nacalai Tesque Inc. cat. 36421-35) per well.

### (2) PCR conditions

A PCR reaction mixture was determined to contain 1 µl of each of the above PCR samples, 5 µl of 10 × LA buffer II, 5 µl of MgCl₂ (25 mM), 5 µl of dNTP (2.5 mM), 2 µl of each primer (10 µM each), 0.5 µl of LA Taq (5 IU/µl and cat. RR002B), and 29.5 µl of distilled water (total 50 µl). For screening of the cells in which the KO1 vector had been transfected, TP-F4 and THygro-R1 were used as PCR primers. For screening of the cells in which the KO2 vector had been introduced, TP-F4 and THygro-F1 were used as PCR primers.

Moreover, PCR conditions employed for the cells into which the KO1 vector had been transfected consisted of pre-heating at 95°C for 1 minute, 40 amplification cycles (each cycle consisting of 95°C for 30 seconds, 60°C for 30 seconds, and 60°C for 2 minutes), and additional heating at 72°C for 7 minutes. PCR conditions employed for screening of the cells into which the KO2 vector had been transfected consisted of pre-heating at 95°C for 1 minute, 40 amplification cycles (each cycle consisting of 95°C for 30 seconds and 70°C for 3 minutes), and additional heating at 70°C for 7 minutes.

Primers are as shown below. In cell samples of homologous recombinants, an approximately 1.6 kb DNA in the case of the KO1 vector and an approximately 2.0-kb DNA in the case of the KO2 vector were amplified. Regarding the primers, TP-F4 was set in the 5' region of fucose transporter genome outside the vector. THygro-F1 and THygro-R1 were set within Hyg^{r} in the vector.
Forward primer (KO1, KO2)
TP-F4 5'-GGA ATG-CAG CTT CCT CAA GGG ACT CGC-3' (SEQ ID NO: 8)
Reverse primer (KO1)
THygro-R1 5'-TGC ATC AGG TCG GAG ACG CTG TCG AAC-3' (SEQ ID NO: 9)
Reverse primer (KO2)
THygro-F1 5'-GCA CTC GTC CGA GGG CAA AGG AAT AGC-3' (SEQ ID NO: 10)

### 5. PCR screening results

918 cells into which the KO1 vector had been transfected were analyzed. Of these -cells, 1 cell was considered to be a homologous recombinant (with homologous recombination efficiency of approximately 0.1%). Furthermore, 537 cells into which the K02 vector -had been transfected were analyzed. Of these cells, 17 cells were considered to be homologous recombinants (with homologous recombination efficiency of approximately 3.2%).

### 6. Southern blot analysis-

Such confirmation was also performed by the Southern blot method. 10 µg of genomic DNA was prepared from the cultured cells according to a standard method, and then Southern blot was performed. A 387-bp probe was prepared from the region ranging from No. 2,113 to No. 2,500 of the nucleotide sequence shown in SEQ ID NO: 1 by the PCR method using the following 2 primers. The thus prepared probe was used for confirmation by the Southern blot method. The genomic DNA was digested with *Bgl* II.
Forward primer
Bgl-F: 5'-TGT GCT GGGAAT TGA ACC CAG GAG-3' (SEQ ID NO: 11)
Reverse primer
Bgl-R: 5'-CTA CTT GTC TGT GCT TTC TTC C-3' (SEQ ID NO: 12)

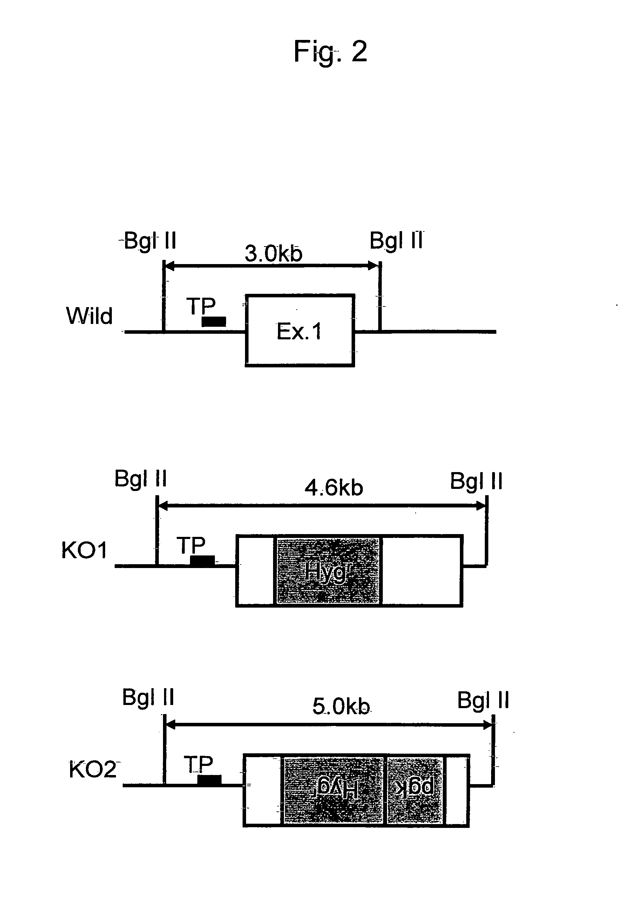

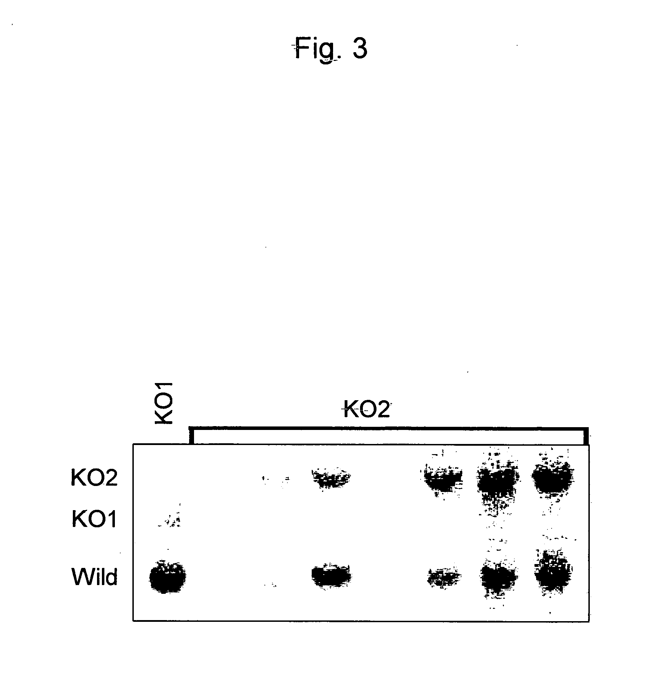

As- a result of digestion with *Bgl* II, an approximately 3.0-kb band was detected from the -chromosome of the fucose transporter, an approximately 4.6-kb band was detected from the chromosome into which the KO1 vector had been inserted after homologous recombination, and an approximately 5.0-kb band was detected from the chromosome into which the KO2 vector had been inserted after homologous recombination. Fig. 2 shows the band patterns after digestion with Bgl II following the knockout of the first step. Fig. 3 shows data of Southern blot. 1 cell line of homologous recombinants using the KO1 vector and 7 cell lines of homologous recombinants using the KO2 vector were used for this experiment. The only one cell line obtained from the KO1-vector tarnsfection was named 5C1. Subsequent analysis revealed that this cell line is composed of multiple cell populations. Cloning was performed by the limiting dilution method for use in the subsequent experiments. Furthermore, the cell line obtained from the KO2-vector transfection was named- 6E2.

### 7. Second step of knock out

3 types of vector were used for homologous recombinants obtained from transfection of the KO1 vector or the KO2 vector, so as to attempt to -establish cell lines completely lacking the fucose transporter gene. Combinations of such vectors with cells are as follows: method 1 (KO2 vector and 5C1 cells (KO1)); method 2 (KO2 vector and 6E2 cells (K02)); and method 3 (KO3 vector and 6E2 cells (KO2)). The vectors were separately transfected into each cell type. At 24 hours after vector introduction, selection was initiated using hygromycin B or puromycin (Nacalai Tesque Inc., cat. 29455-12). Hygromycin B was used in the case of method 1 at a final concentration of 1 mg/ml and in the case of method 2 at a final concentration of 7 mg/ml. Further, in the case of method 3, hygromycin B was added to a final concentration of 0.15 mg/ml and puromycin was added to a final concentration of 8 µg/ml.

### 8. Screening of homologous recombinants by PCR

Samples were prepared as described above. Screening in the case of method 1 was performed by PCR to detect signals of homologous -recombination by both KO1 vector and KO2 vector. For screening in the case of method 2, the following PCR primers were designed. TPS-F1 was set in the Nos. 3,924-to-3,950 region and SHygro-R1 was set in the Nos. 4,248-to-4,274 region of the nucleotide sequence shown in SEQ ID NO: 1. With the use of these PCR primers, a gene region of 350 bp of the fucose transporter, which is deficient due to the KO2 vector, is amplified. Therefore, for PCR screening in the case of method 2, cells in which such 350-bp region had not been amplified were determined to be cells completely lacking the fucose transporter gene. PCR conditions consisted of preheating at 95°C for 1 minute, 35 amplification cycles (each cycle consisting of 95°C for 30 seconds and 70°C for 1 minute), and additional heating at 70°C for 7 minutes.
Forward primer
TPS-F1: 5'-CTC GAC TCG TCC CTA TTA GGC AAC AGC-3' (SEQ ID NO: 13)
Reverse primer
SHygro-R1: 5'-TCA GAG GCA GTG GAG CCT CCA GTC AGC-3' (SEQ ID NO: 14)

In the case of method 3, TP-F4 was used as a forward primer and RSGR-A was used as a reverse primer. PCR conditions consisted of preheating at 95°C for 1 minute, 35 amplification cycles (each cycle consisting of 95°C for 30 seconds, 60°C for 30 seconds, and 72°C for 2 minutes), and additional heating at 72°C for 7 minutes. In the cell samples of homologous recombinants in the case of the KO3 vector, an approximately 1.6-kb DNA was amplified. As a result of the PCR, homologous recombination by the KO3 vector was confirmed. It was also confirmed that the region of homologous recombination-by the KO2 vector was remained.

### 9. Results -of PCR screening

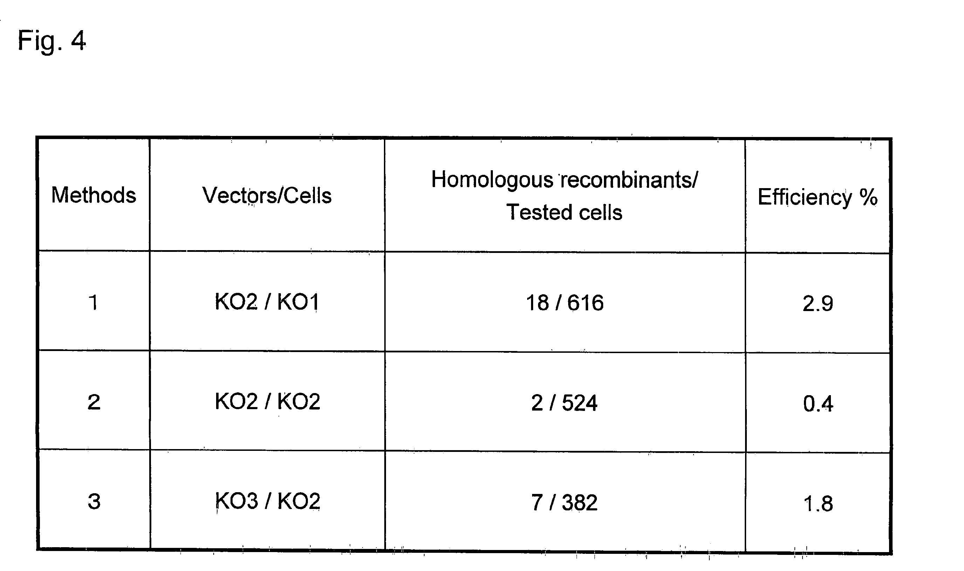

In the case of method 1, 616 cells were analyzed. Of these cells, 18 cells were thought to be homologous- recombinants (homologous recombination efficiency : 2.9%) (Fig. 4). In the case of method 2, 524 cells were analyzed. Of these cells, 2 cells were thought to be homologous recombinants (homologous recombination efficiency: approximately 0.4%). Furthermore, in the case of method 3, 382 cells were analyzed. Of these cells, 7 cells were thought to be homologous recombinants (homologous recombination efficiency: approximately 1.8%).

### 10. Southern blot analysis

Southern blot analysis was performed according to the above methods. As a result, among the analyzed cells, one cell completely lacking the fucose transporter gene was discovered. In the case of knockout of -the first step, PCR analysis results were consistent with Southern blot analysis results. Regarding knockout of the second step, the two were not consistent with each other. Possible causes of such results are: 1. a mixture of homologous recombinants of either KO1 alone or KO2 alone in the case of method 1; 2. presence of -not a pair of fucose transporter genes (2 fucose transporter genes), but a plural number of pairs of fucose transporter genes (or 3 or more fucose transporter genes); and 3. increased copy number of the fucose transporter gene that had remained after subculture of cell lines in which the knockout of the first step had been successfully performed.

### 11. Fucose expression analysis

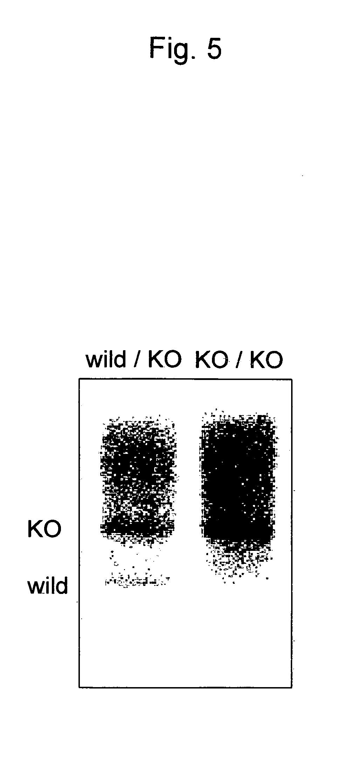

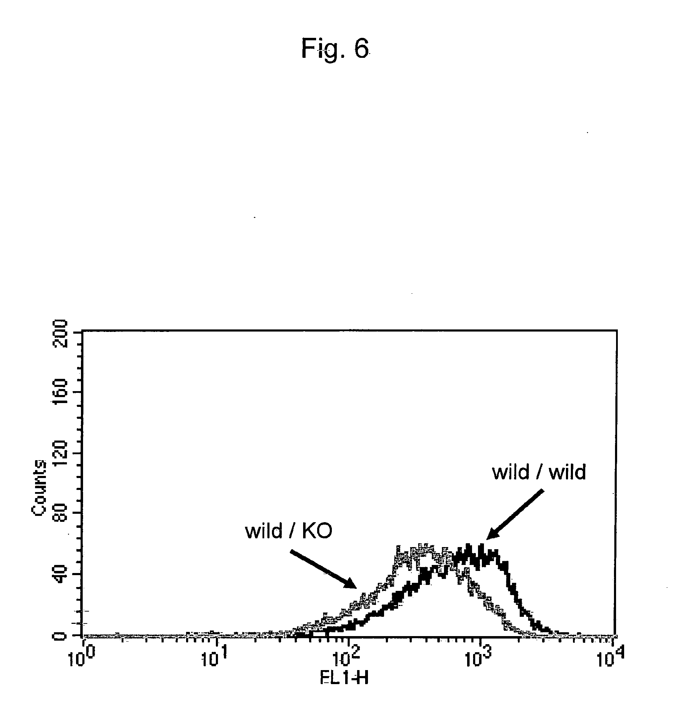

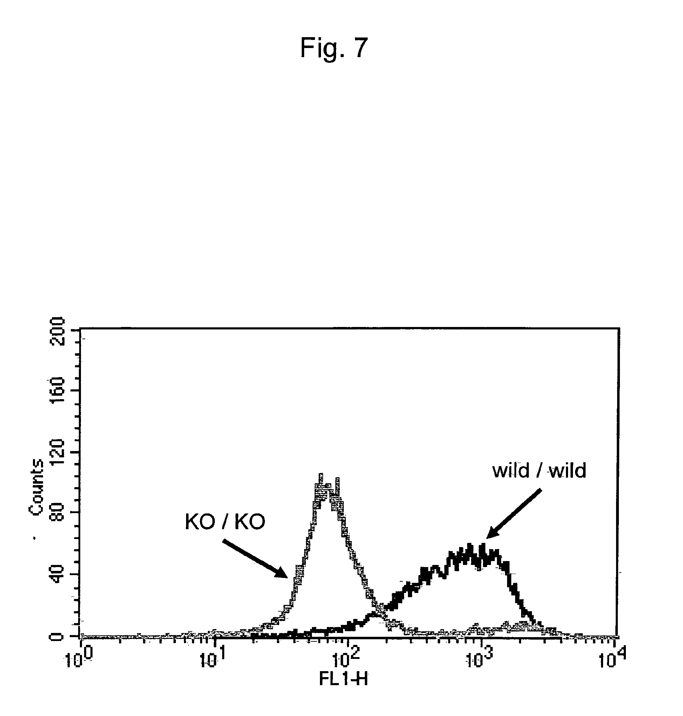

Furthermore, fucose expression in 26- cells that were determined as homologous recombinants by PCR was analyzed. 1×10⁶ cells were stained with 100 µl of PBS (hereinafter, referred to as FACS solution) containing 5 µg/mL Lens culinaris Agglutinin, FITC Conjugate (Vector Laboratories cat. FL-1041), 2.5% FBS, and 0.02% sodium azide for 1 hour during cooling with ice. Subsequently, the cells were washed three times with FACS solution and then analyzed by FACS Calibur (Becton, Dickinson and Company). As a result, it was revealed that only the cells completely lacking the fucose transporter gene as determined by Southern blot analysis exerted decreased fucose expression levels. Figs. 5 to 7 show the results of Southern blot analysis performed for the obtained clones (Fig. 5) and the results of determining fucose expression levels. Fig. 6 shows the results for wild/KO. Fig. 7 shows the results for KO/KO.

The following was demonstrated by the above results.

Only one cell line completely lacked the fucose transporter gene and the number of such cells screened for was 616. Specifically, homologous recombination frequency was approximately as low as 0.16%. Regarding the knockout of the second step, there are several possible reasons for the inconsistency between PCR analysis results and Southern blot analysis results, as described above. However in the case of method 3, since selection was performed using 2 types of drug, it was unlikely that the obtained cell lines would contain homologous recombinants by both the KO2 vector alone and the KO3 vector alone. Moreover, it was unlikely that any of the other cell lines determined as homologous recombinants by PCR would be composed of a plurality of cell populations. The presence of 3 or more fucose transporter genes as described above makes targeting in cells significantly difficult. In such case, it was concluded that homologous recombinants may be impossible to obtain unless the KO1 vector, with which Hyg^{r} is weakly expressed, is used and screening would be performed many times.

All publications, patents, and patent applications cited herein are incorporated herein by reference in their entirety.

## Claims

1. A cell, in which the expression of fucose transporter genes on both chromosomes is artificially suppressed.

2. The cell according to claim 1, in which the fucose transporter genes are disrupted.

3. The cell according to claim 1 or 2, which is an animal cell.

4. The cell according to claim 3, in which the animal cell is a Chinese hamster cell.

5. The cell according to claim 4, in which the animal cell is a CHO cell.

6. The cell according to any one of claims 2 to 5, in which gene disruption is carried out by homologous recombination using a gene targeting vector.

7. The cell according to any one of claims 1 to 6, in which a gene encoding a foreign protein is introduced.

8. The cell according to claim 7, in which the gene encoding the foreign protein is a gene encoding an antibody.

9. A method of producing a protein, comprising culturing the cell according to any one of claims 1 to 8.

10. The production method according to claim 9, in which the protein is an antibody.

11. The production method according to claim 10, comprising producing a protein to which fucose is not added.

12. A method for inhibiting the addition of fucose to a protein, comprising artificially suppressing the expression of fucose transporter genes on both chromosomes upon production of a recombinant protein using a cell.

13. The method for inhibiting the addition of fucose to a protein according to claim 12, comprising artificially suppressing gene expression through deletion of a gene.

14. The method for inhibiting the addition of fucose to a protein according to claim 12 or 13, in which the protein is an antibody.

15. The method for inhibiting the addition of fucose to a protein according to any one of claims 12 to 14, in which the cell is a CHO cell.
